# FASCICULE DE BREVET EUROPEEN

(11) **EP 2 068 619 B1**
(45) Date de publication et mention de la délivrance du brevet: **01.08.2012**
(21) Numéro de dépôt: 07803309.9
(22) Date de dépôt: 06.09.2007
(51) Int. Cl.: A01N 1/02, B01L 3/00

(54) **PROCEDE DE CONDITIONNEMENT DE CELLULES EN CULTURE, ET CONDITIONNEMENT DE CELLULES CORRESPONDANT.**
VERFAHREN ZUR VERPACKUNG VON KULTIVIERTEN ZELLEN UND ENTSPRECHENDE VERPACKTE ZELLEN.
METHOD FOR PACKAGING CELLS IN CULTURE AND CORRESPONDING PACKAGED CELLS.

(30) Priorité: 13.09.2006 FR 0608019
(43) Date de publication de la demande: 17.06.2009
(73) Titulaire: Sarl Biopredic International, 35000 Rennes (FR)
(72) Inventeur: CHESNE, Christophe, 56000 Vannes (FR)
(74) Mandataire: Larcher, Dominique
(86) Numéro de dépôt international: PCT/EP2007/059355
(87) Numéro de publication internationale: WO 2008/031759

(56) Documents cités:
- EP-A- 1 085 081
- FR-A1- 2 682 980
- FR-A1- 2 689 139
- US-A1- 2005 235 368

## Description

Le domaine de l'invention est celui du conditionnement de cellules en culture en vue de leur transport. Plus précisément, l'invention concerne un procédé pour conditionnement des cellules en milieu de culture contenu dans des récipients, à savoir des puits portés par des plaques.

La commercialisation de cellules sous forme de plaques de culture prêtes à l'emploi nécessite une phase de transport entre le producteur et/ou le distributeur et l'utilisateur final. Les plaques de culture ne peuvent être transportées telles quelles car aucun système n'est prévu pour empêcher que le milieu de culture ne sorte des puits, par simple renversement. Or, les cellules ne peuvent survivre pendant le transport sans être au contact direct et permanent du milieu de culture.

Différentes solutions techniques sont utilisées pour cette étape de transport.

La solution technique la plus fréquemment utilisée, et la moins onéreuse, consiste à recouvrir les plaques remplies de milieu de culture d'un film plastique adhésif. L'utilisation des films adhésifs pour étanchéifier les plaques de culture se heurte à différents types de problèmes, tels que le relargage de produits potentiellement toxiques.

Par ailleurs, un volume résiduel formant bulle d'air est le plus souvent présent dans les puits (le remplissage à 100% des puits étant très difficile à obtenir). Ces bulles d'air piégées sous le film doivent être chassées manuellement à l'aide d'une seringue, ceci pour chaque puits : les bulles d'air peuvent en effet se retrouver au contact de cellules lors du transport, notamment en cas de retournement des plaques de cellules en monocouche, et sont susceptibles de provoquer l'endommagement ou la mort des cellules. Il faut alors recouvrir la plaque d'un second film plastique adhésif pour boucher les trous faits par la seringue. Ce processus de préparation des plaques pour le transport présente donc l'inconvénient d'être long et donc peu productif en terme industriel.

En outre, la température externe durant le transport est susceptible de varier considérablement, notamment lors des transports lointains par avion. Ceci provoque des dilatations et rétractions des matériaux de conditionnement, qui ont pour effet leur perte d'étanchéité, ce qui a pour conséquence la fuite du milieu de culture hors des puits des plaques multi-puits, celles-ci n'étant pas scellées. De plus, l'utilisation de films adhésifs pour le transport de plaques présentant des puits de grande tailles (plaques à 6 puits notamment) ne permet pas d'assurer l'étanchéité de ceux-ci, car le poids du liquide des puits est supérieur à la force d'adhésion du film adhésif.

Il existe aussi des films thermoscellables, dont la force d'adhésion est supérieure aux adhésifs, mais la soudure est peu réversible et laisse des traces au déscellage, ce qui est mal accepté par le client final.

D'autres systèmes de bouchage des plaques multi-puits sont utilisés, comme les bouchons plastiques ou caoutchouc à placer sur les puits. Les inconvénients de ce système sont également nombreux. Tout d'abord, le prix de revient de ce système de bouchage est élevé, ce qui le rend peu utilisable dans ce secteur industriel. Mais la limite principale de ces systèmes de fermeture par des bouchons est leur mauvaise étanchéité du fait des variations de cote des puits dans une même plaque ou d'un lot de plaques à l'autre. Pour résoudre ce problème, on peut utiliser des bouchons souples, mais dans ce cas la présence de bouchons sur les puits réduit de 2/3 le volume de milieu de transport dans chaque puits. Ce volume de milieu disponible au-dessus des cellules est insuffisant pour assurer l'alimentation en matière nutritive et pour assurer une stabilité de pH tout au long du transport, celui-ci pouvant durer 3 jours.

Un autre inconvénient est qu'il faut un modèle de bouchon par type de plaque, alors qu'une solution unique, s'adaptant à tous types de support, serait plus avantageuse en termes industriels.

On connaît aussi par la demande de brevet FR 2862980 déposée au nom de la demanderesse une technique consistant à rendre le milieu de transport solide, par l'ajout de poudre de polymères de polysaccharides. L'ajout de ces grains de polymères au milieu de culture a pour effet de former un hydrogel compact sur les cellules, contournant ainsi le problème des fuites grâce à une solidification du milieu de transport. En pratique, il s'est avéré que ce système demandait une mise au point importante pour garantir la neutralité de l'hydrogel sur chaque type cellulaire différent. D'autre part l'aspect des grains de polymères était difficilement accepté par l'utilisateur final.

Un autre système de transport, décrit dans la demande de brevet US 2005/0235368 a été développé pour le transport d'un type particulier de cellules : les ovocytes transgéniques de *Xenopus laevis.* Selon cette méthode de transport, l'étanchéité est effectuée par la pression d'une plaque de silicone sur les puits.
Cette pression est appliquée par compression.

On connaît également par la demande de brevet français FR 2689139 un kit de transport de cellules immobilisées dans des particules de gel. Le kit consiste en une plaque support comportant une pluralité de cuvettes comprenant les cellules immobilisées ainsi qu'un couvercle d'étanchéité comprenant des cavités dont la forme, la dimension et la position permettent d'assurer une fermeture étanche des cuvettes Enfin, une plaque absorbante vis-à- vis du milieu de culture est placée de manière à entourer les cuvettes au moment de son montage sur la plaque support. Au moment du transport, on remplit les cuvettes de milieu de culture. Le positionnement du couvercle fait remonter le milieu le long des parois entre la cuvette et la cavité, ce qui a pour effet de chasser l'air et d'éviter le dessèchement des cultures.

Aucune de ces solutions techniques ne résout définitivement les problèmes de la phase de transport longue distance, soit celui des fuites constatées de façon plus ou moins fréquentes, soit celui du volume de milieu de transport (solution bouchons).

L'invention a pour objectif de pallier les inconvénients de l'art antérieur.

Plus précisément, l'invention a pour objectif de proposer une technique de conditionnement de cellules en cultures destinées à être transportées, qui soit plus fiable que les techniques de l'art antérieur, en particulier en termes de préservation de la qualité des cellules.

L'invention a également pour objectif de fournir une telle technique qui permette de remplir les récipients en milieu de culture avec un taux de remplissage important.

L'invention a aussi pour objectif de fournir une telle technique qui assure une étanchéité satisfaisante des récipients.

Un autre objectif de l'invention est de proposer une telle technique qui soit utilisable avec tout type de plaque ou quasiment.

Encore un autre objectif de l'invention est de fournir une telle technique qui soit facile et peu onéreuse à mettre en oeuvre.

Ces objectifs, ainsi que d'autres qui apparaîtront par la suite, sont atteints grâce à l'invention qui a pour objet un procédé de conditionnement de cellules en culture en vue de leur transport, selon la revendication 1

Ainsi, l'invention propose une solution technique permettant de garantir l'étanchéité, non pas par une méthode de compression comme dans le brevet US 2005/0235368, mais par une méthode de dépression, réalisée par le moyen d'un ensachage sous vide partiel des plaques de culture.

Une telle technique est utilisable avec tout type de plaque couramment utilisée, ces plaques pouvant indifféremment présenter 6 à 3456 puits.

On note que la solution technique proposée par l'invention vise à sceller les puits, le procédé de scellage sous vide devant être réversible.

Cette solution va à l'encontre des pratiques du domaine technique, dans la mesure où il est connu que les cultures de cellules ne peuvent pas supporter une mise sous vide poussé (c'est-à-dire pression finale proche de 0 Bar), ces conditions aboutissant à une ébullition du milieu de transport des cellules, qui a pour conséquence d'altérer la viabilité des cellules. C'est d'ailleurs la raison pour laquelle aucun homme de l'art n'avait préalablement envisagé ce procédé visant l'étanchéification par dépression.

En effet, lors de la diminution de la pression dans les puits de culture, on constate un « bouillonnement » du milieu de transport au-dessus des cellules. Ce bouillonnement est dû à un dégazage rapide du milieu de transport. Les gaz dissous dans ces milieux à pression atmosphérique vont en partie retourner à l'état gazeux lors de la diminution de la pression (O₂, CO₂, N₂ principalement).

Il est donc d'une importance cruciale d'éviter ce dégazage.

Outre le fait qu'un tel bouillonnement génère des éclaboussures, il modifie l'équilibre acido-basique du milieu et des cellules cultivées.

Cet équilibre acido-basique est en effet réalisé par un tampon carbonate-bicarbonate, qui permet de solubiliser le CO₂ selon la réaction suivante : CO₂ + H₂0 donne HC03- + H⁺

Le dégazage, même partiel, de la solution, provoque une alcalinisation du milieu, toxique pour les cellules.

On note que la mise en ébullition peut être entraînée par d'autres phénomènes physico-chimiques, provoqués par la combinaison des paramètres de pression, de volume et de température.

En tout état de cause, la formation de bulles est susceptible de provoquer le décrochage des cellules du puits, qui reviennent alors en suspension dans le milieu. Dans ce cas, au moment de l'ouverture par l'opérateur, le retrait du milieu pour accéder aux cellules (censées être au fond des puits) entraîne la perte des cellules.

En agissant sur les paramètres de température et de remplissage selon différents modes de réalisation qui vont être explicités par la suite, l'invention propose une solution technique pour éviter cette formation de bulles, ce qui permet de respecter l'équilibre acido-basique des cellules, de travailler dans des conditions de propreté conformes à la culture cellulaire, et d'éviter que l'humidification (par des éclaboussures dues à l'ébullition) des bords des puits (récipients) ne gêne la fermeture étanche de ceux-ci.

La solution technique selon l'invention est double : agir sur la température et sur le volume de milieu, ou plus exactement le volume de gaz laissé au-dessus du milieu dans les puits.

Préférentiellement, ladite phase de remplissage est précédée d'une étape de mise en température dudit milieu de culture de façon que ledit milieu de culture présente une température correspondante auxdites conditions de température.

Selon une solution avantageuse, ladite étape de mise en dépression est réalisée de façon à obtenir une pression dans ledit récipient comprise entre environ 20 millibars et environ 120 millibars après ladite étape de fermeture, et de façon préférée entre toutes pour obtenir une pression d'environ 60 millibars.

On constate en effet que lorsque le vide est trop poussé, en dessous d'environ 20 millibars, les cellules commencent à manifester des signes de souffrance et qu'au-dessus de 120 millibars, le vide est insuffisant pour assurer une pression homogène sur des éléments de fermeture des puits, et ne pourra pas garantir l'absence de fuites.

Selon une première approche de l'invention lesdits récipients sont remplis sensiblement complètement dudit milieu de culture et ladite étape de mise en dépression est conduite tandis que ledit milieu de culture présente une température supérieure à 0° C et inférieure à environ 20°C, et préférentiellement tandis que ledit milieu de culture présente une température d'environ 4°C.

Ainsi, la diminution de la température du milieu retarde le moment du dégazage lors de la diminution de la pression extérieure.

On note que, en dessous de 0°C les cellules gèlent, et au-dessus de 20°C le phénomène d'ébullition devient trop important.

Selon une deuxième approche de l'invention ladite étape de mise en dépression est conduite tandis que ledit milieu de culture présente une température supérieure à environ 20° C et inférieure à environ 37°C, lesdits récipients étant remplis par ledit milieu de culture avec un taux de remplissage compris entre environ 70% et environ 95%, et préférentiellement avec un taux de remplissage d'environ 90%.

En dessous de 70% de remplissage, on constate qu'une grosse bulle d'air reste dans les puits à l'issue du procédé d'emballage, cette bulle d'air pouvant endommager la viabilité de la culture si un contact prolongé avec le tapis cellulaire se produit au cours du transport.

Au-dela de 95% de remplissage, le milieu déborde lors du procédé d'emballage. En conséquence de cet ajustement du volume de remplissage, le dégazage provoqué par la chute de pression à laquelle est soumis le système de conditionnement sera atténué par la présence d'une faible quantité d'air dans les puits (10% environ).

Ceci a pour effet de limiter le bouillonnement du milieu, et ainsi de préserver l'intégrité du tapis cellulaire.

Selon l'invention, ladite étape de fermeture comprend une étape de mise en place sur ledit récipient d'un matériau de fermeture susceptible de se déformer.

Un tel matériau déformable peut donc épouser la forme du puits et épouser parfaitement le contour de celui-ci, ce qui assure une parfaite étanchéité de la fermeture.

Dans ce cas, ledit matériau de fermeture présente avantageusement une dureté moyenne de 60 shores, ledit matériau de fermeture étant préférentiellement un matériau élastomère.

La déformabilité et la souplesse du matériau élastomère sont importants, cette plaque ne devant être ni trop rigide, car elle épouserait mal la forme des puits, ni trop souple, ce qui provoque une déformation lors de la mise sous vide du système, cette déformation étant susceptible de créer des fuites.

Selon une solution préférée, le procédé comprend une étape de recouvrement dudit matériau de fermeture par un matériau rigide de maintien.

Le matériau de fermeture étant en matière souple, celui-ci peut avoir tendance, dans certains cas, à se déformer dans les puits, au risque qu'il présente des hétérogénéités autour des puits, ce qui avait pour effet de favoriser les fuites.

La plaque de matériau plastique rigide placée au dessus de la plaque élastomère a pour rôle d'apporter une certaine rigidité au système de fermeture, et de permettre à la pression créée par la diminution de pression atmosphérique de se répartir de façon homogène sur l'ensemble de la plaque de culture.

Cette plaque permet également d'obtenir une meilleure préservation des cellules, grâce à son imperméabilité aux gaz.

L'invention concerne également un conditionnement selon la revendication 10

Préférentiellement, le conditionnement comprend une plaque de matériau rigide de maintien recouvrant ledit matériau de fermeture du récipient.

D'autres caractéristiques et avantages de l'invention apparaîtront plus clairement à la lecture de la description suivante de deux modes de réalisation préférentiels de celle-ci, donnés à titre d'exemples illustratifs et non limitatifs, et des dessins annexés parmi lesquels :
- la figure 1 est une représentation synoptique d'un procédé selon l'invention ;
- la figure 2 est une représentation schématique d'un conditionnement réalisé par un procédé selon l'invention.

Tel qu'indiqué précédemment, le principe de l'invention réside dans le fait de créer une dépression autour du système de conditionnement pour le transport qui, en exerçant de façon indirecte une pression forte, constante et répartie de façon homogène sur les moyens de fermeture, assure le transport des plaques sans fuite de milieu.

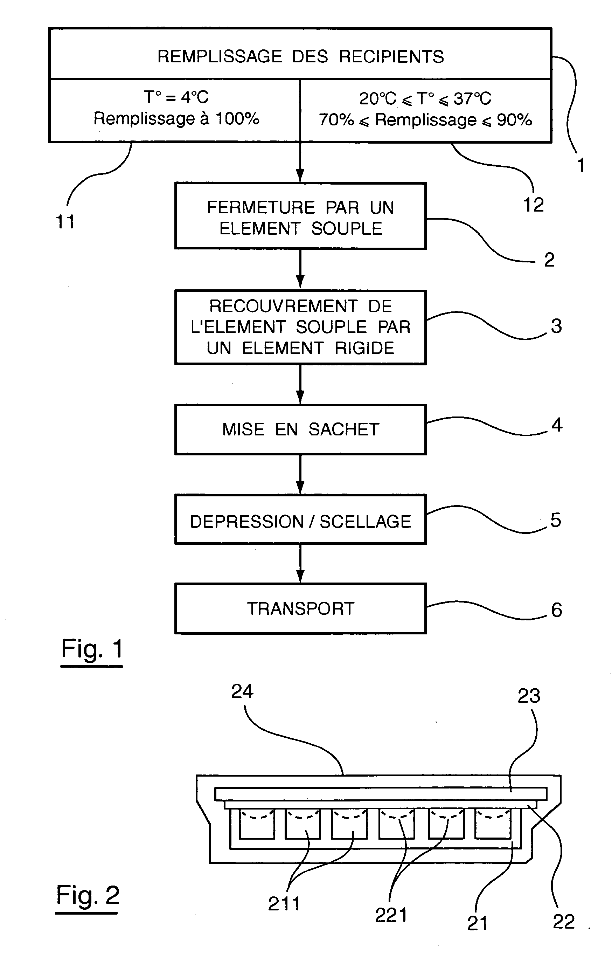

En référence à la figure 1, la première étape 1 consiste à réaliser un remplissage des récipients selon un taux déterminé explicité ci-après.

La fermeture des puits comprend une étape 2 consistant à rapporter sur les puits un matériau élastomère présentant préférentiellement une dureté de 60 shores.

Ce matériau est stérilisé par les techniques usuelles de stérilisation avant d'être utilisé.

Lors de l'étape 3, un matériau rigide de maintien, préférentiellement un matériau transparent rigide, est rapporté sur la plaque élastomère.

L'étape 4 suivante concerne la mise en sachets des récipients fermés par les plaques élastomère et rigide.

Lors de l'étape 5, on réalise une aspiration de l'air contenu dans les sachets. Un scellage du sachet est effectué tandis que la dépression de l'intérieur du sachet est réalisée, ceci par une technique connue en elle-même.

Les paramètres de pression sont maîtrisés de façon fme par l'appareil chargé de réaliser le vide.

Différents appareils permettent de faire le vide avec une bonne précision de réglages ,dans un cycle de 20 secondes de faire un vide très précis et de souder un sachet lors de l'étape 5 mentionnée précédemment.

La fermeture de chacun des puits est donc réalisée par pression d'une plaque élastomère sur les rebords des puits. L'étanchéité est assurée lorsque la dépression créée par le vide exerce une force suffisante pour solidariser la plaque élastomère avec les bords de puits, ceci par l'intermédiaire des parois du sachet.

Lors d'essais réalisés, la formation d'un bouillonnement du milieu de transport à 37°C lors de la mise sous vide à 60 millibars a rapidement été mise en évidence. L'ébullition en générant des éclaboussures sur les bords de puits empêche le contact sec étanche de la plaque élastomère sur les bords du puits. Le milieu contenu dans le puits passe alors par capillarité hors du puits dès que l'on secoue la plaque un peu fort. L'étanchéité du système n'est plus assurée.

Il semble que cette ébullition ou ses effets puissent être limités pour une même force de vide, par :
- un remplissage partiel du puits ; ou
- un refroidissement des milieux de culture à 4°C.

Dans le cas d'un remplissage partiel (correspondant à l'étape 12), le milieu de culture est préchauffé entre 20°C et 37°C (préférentiellement 37°C), le remplissage du puits étant réalisé avec un taux de remplissage compris entre 70% et 95%, préférentiellement 90%.

L'inconvénient du remplissage partiel est que la capacité du conditionnement à conserver le tapis cellulaire devient limitée en cas de retournement de la plaque pendant le transport. En effet la bulle d'air présente dans le puits est de taille importante et peut, dans certain cas assécher le tapis cellulaire en cas de contact prolongé.

Dans le cas d'un milieu refroidi à 4°C (correspondant à l'étape 11), les puits sont remplis avec 3.25 ml de milieu de culture (=100% du volume de liquide qu'il est possible d'introduire dans un puits, en tenant compte de la présence d'un ménisque).

Lors de la mise sous vide a 60 millibars, le milieu refroidit à 4°C ne « bouillonne » pas, le point d'ébullition est donc abaissé.

Il n'y a plus d'éclaboussures et plus de problème de contact a l'air en cas de retournement prolongé de la plaque de culture.

Les résultats des essais sont reportés dans le tableau 1 ci-après.

**Tableau 1**

| Vide (millibars) | Température du milieu de culture | Niveau de remplissage du puits (%) | 'Ebullition' | Etanchéité assurée |
|---|---|---|---|---|
| 60 | 37°C | 100% (=3.25ml) | oui | **non** |
| 60 | 17°C | 75%(=2.40ml) | oui | **oui** |
| 60 | 4°C | 100% (=3.25ml) | non | **oui** |

Dans le cadre des présents exemples de réalisation, il a été confirmé que l'étanchéité est assurée pour un niveau de vide égal ou supérieur à 60 millibars (60 → 0 millibars). On a cependant noté une déformation importante de l'élément de fermeture élastomère au delà de 40 millibars (40 → 0 millibars). Pour un niveau vide moins poussé ( inférieur à 60 millibars et jusqu'à presque la pression atmosphérique), des fuites de milieu de culture ont été observée dès que l'on secoue la plaque de culture un peu fort.

Des essais ont été réalisés sur des cultures primaires d'hépatocytes en plaques de 24 puits.

Ces cellules ont été choisies pour la mise au point de ce nouveau système de transport car elles sont parmi les plus sensibles (fragiles). En déterminant des conditions idéales pour ces cellules, ces conditions sont optimisées pour d'autres types cellulaires plus résistants comme les lignées.

Les cultures primaires d'hépatocytes ont été conditionnées sous vide pendant 72h avant d'être remises en culture selon les conditions habituelles. Les observations morphologiques sont reportées dans le tableau 2 ci-après.

**Tableau 2 :**

| **Condition** | **Vide (millibars)** | **Température du milieu de culture** | **Niveau de remplissage du puits(%)** | **Qualité de la plaque élastomère** | **Ajout de plaque de maintien** | **Etanchéité assurée** | **Aspect des cellules** |
|---|---|---|---|---|---|---|---|
| 1 | 60 | 37°C | 75% (=2,40ml) | Elastomère épaisseur 4 mm 86x128 mm | non | oui | Belle morphologie hépatique, Présence de cellules mortes |
| 2 | 60 | 37°C | 75% (=2.40 ml) | Elastomère épaisseur 3 mm, 86 x 128mm | non | oui | Idem ci-dessus |
| 3 | 60 | 37°C | 75% (=2.40 ml) | Elastomère alimentaire, épaisseur 1mm, 86 x 128mm | non | oui | Plaque élastomère très déformé, dépression du silicone dans chaque puits, cellules fragilisées |
| 4 | 60 | 37°C | 75% (=2.40 ml) | Elastomère épaisseur 4 mm, 86 x 128 mm | oui | oui | Très belle morphologie hépatique, moins de cellules à se décoller (=cellules mortes) |
| 5 | 60 | 4°C | 100% (=3.25 ml) | Elastomère épaisseur 4 mm, 86 x 128 mm | oui | oui | Très belle Morphologie hépatique, moins de cellules à se décoller (=cellules mortes) |

L'ajout de la plaque de maintien rigide pour limiter la déformation de la plaque élastomère et optimiser son positionnement sur les puits a également été étudiée.

On note d'après les résultats reportés dans le tableau 2 que la plaque rigide de maintien améliore les résultats en termes de préservation des cellules, ceci grâce à son imperméabilité au gaz (ce qui n'est pas le cas de la plaque élastomère seule).

Deux types de sachets ont été testés pour réaliser l'emballage externe :
- un sachet épais ;
- un sachet souple .

Lors de l'emballage sous vide (60 millibars), l'aspiration complète de l'air est mieux réalisée dans un sachet souple.

En effet dans un sachet plastique épais et rigide, l'air reste piégé au niveau des plis formés à chaque angle de la plaque de culture, l'aspiration n'est pas complète. La fermeture des récipients par dépression est par conséquent moins fiable.

Des cultures primaires d'hépatocytes ont été conditionnées sous-vide en reprenant chacun des meilleurs paramètres déterminés précédemment :
- vide effectué a 60 millibars
- remplissage du volume du puits a 100%, avec un milieu de transport refroidi a 4°C - plaque élastomère de 3 mm épaisseur
- ajout d'une plaque rigide
- sachet souple pour emballage externe

La bonne préservation des cultures primaires d'hépatocytes a été évaluée après 72h de conditionnement (temps de transport maximum nécessaire pour des cellules actuellement au laboratoire)., sur des critères morphologiques et enzymatiques. Les résultats sont représentés dans le tableau 3 ci-après.

**Tableau 3:**

| | | Morphologie | Nifedipine oxidase activity (CYP3A4/5)(nanomole /hr/mgproteins): | Phenacetin O-deethylase activity (CYP1 A2)(nanom ole/hr/mgproteins) | Paracetamol glucuronidation activity(nanomole/ hr/mgproteins) | Paracetamol sulfation activity(nanomole/hr/m g proteins): |
|---|---|---|---|---|---|---|
| Culture primaire n° 1 | Témoin dans incubateur 37°C, 95/5 air/C02 | Cell réfringentes, noyaux et mb bien visibles, peu de cell mortes | **2.1** | **0.5** | **3.7** | **1.6** |
| | Conditionnée 72h sous vide, Remise en culture dans incubateur 37°C, 95/5 air/C02 | Idem témoin | **1.8** | **0.6** | **4.5** | **2.0** |
| Culture primaire n°2 | Témoin dans incubateur 37°C, 5% C02 | Cell réfringentes, noyaux et mb bien visibles | **3.0** | **0.2** | **5.8** | **3.9** |
| | Conditionnée 72h sous vide, Remise en culture dans incubateur 37°C, 95/5 air/C02 | Idem témoin | **1.6** | **0.2** | **6.8** | **3.9** |

Lors de l'étape 5 de transport, les plaques de culture ainsi conditionnées sont expédiées selon les usages de la profession. Selon ces usages, la température externe doit être contrôlée. Le système sera en outre placé dans une boîte isotherme de polyuréthane. Ces usages ne font pas l'objet de l'invention.

A réception, les utilisateurs finaux doivent rompre le vide pour récupérer les plaques de culture. Ceci se fait sans précaution particulière autre que les précautions assurant le maintien de la stérilité. Le sachet sous vide est coupé, par exemple par une simple paire de ciseaux. Un conditionnement obtenu par un procédé selon l'invention est illustré par la figure 2.

Un tel conditionnement se compose :
- d'une plaque 21 présentant une pluralité de puits 211 contenant des cellules en milieu de culture sous vide partiel ;
- une plaque élastomère 22 dont la souplesse lui permet de se déformer vers l'intérieur des puits (ceci étant représenté par les déformations 221 en traits pointillés) ;
- une plaque de maintien 23 (ou plastique rigide transparent) maintenant la plaque élastomère 22 sur les puits ;
- un sachet souple scellé 24.

## Revendications

1. Procédé de conditionnement de cellules vivantes en culture en vue de leur transport puis de leur remise en culture, lesdites cellules étant dans un milieu de culture contenu dans au moins un récipient constitué par au moins un puits porté par un plaque de culture,
**caractérisé en ce qu'**il comprend :
une phase de remplissage dudit au moins un récipient par ledit milieu de culture contenant lesdites cellules vivantes selon un taux de remplissage déterminé ;
une étape de fermeture étanche et sous vide dudit au moins un récipient comprenant :
une mise en place sur ledit au moins un récipient d'un matériau de fermeture susceptible de se déformer ;
une mise en sachet souple dudit au moins un récipient et ;
une mise en dépression de l'intérieur dudit sachet ;
un scellage dudit sachet, la mise en dépression étant maintenue, ladite étape de fermeture étant réalisée dans des conditions de température corrélées audit taux de remplissage en vue d'éviter la mise en ébullition dudit milieu de culture lors de ladite mise en dépression.

2. Procédé de conditionnement de cellules vivantes selon la revendication 1, **caractérisé en ce que** ladite phase de remplissage est précédée d'une étape de mise en température dudit milieu de culture de façon que ledit milieu de culture présente une température correspondante auxdites conditions de température.

3. Procédé de conditionnement de cellules en culture selon l'une des revendications 1 et 2, **caractérisé en ce que** ladite étape de mise en dépression est réalisée de façon à obtenir une pression dans ledit récipient comprise entre environ 20 millibars et environ 120 millibars après ladite étape de fermeture.

4. Procédé de conditionnement de cellules en culture selon la revendication 3, **caractérisé en ce que** ladite étape de mise en dépression est réalisée de façon à obtenir une pression dans ledit récipient d'environ 60 millibars après ladite étape de fermeture.

5. Procédé de conditionnement de cellules en culture selon l'une quelconque des revendications 1 à 4, **caractérisé en ce que** ladite étape de mise en dépression est conduite, tandis que ledit milieu de culture présente une température supérieure à 0° C et inférieure à environ 20°C, ledit au moins un récipient étant rempli sensiblement complètement dudit milieu de culture.

6. Procédé de conditionnement de cellules en culture selon la revendication 5, **caractérisé en ce que** ladite étape de mise en dépression est conduite, tandis que ledit milieu de culture présente une température d'environ 4°C.

7. Procédé de conditionnement de cellules en culture selon l'une quelconque des revendications 1 à 6, **caractérisé en ce que** ledit matériau de fermeture présente une dureté moyenne d'environ 60 shores.

8. Procédé de conditionnement de cellules en culture selon la revendication 7, **caractérisé en ce que** ledit matériau de fermeture est un matériau élastomère.

9. Procédé de conditionnement de cellules en culture selon l'une quelconque des revendications 1 à 8, **caractérisé en ce qu'**il comprend une étape de recouvrement dudit matériau de fermeture par un matériau rigide de maintien.

10. Conditionnement de cellules vivantes en culture destinées à être transportées puis remises en culture, ledit conditionnement incluant au moins un récipient (21) constitué par au moins un puits porté par une plaque de culture fermé de façon étanche par un matériau de fermeture (22) susceptible de se déformer, ledit récipient étant au moins partiellement rempli d'un milieu de culture contenant lesdites cellules vivantes, et un sachet (24) scellé contenant ledit au moins un récipient ou lesdits récipients (21) étant sous vide dans ledit sachet (24).

## Claims

1. Method for packaging living cells in culture in order to transport them and then to return them to the culture, the cells being in a culture medium which is contained in at least one receptacle comprising at least one well carried by a culture plate,
**characterised in that** the method comprises:
a phase for filling the at least one receptacle with the culture medium which contains the living cells in accordance with a predetermined filling level;
a step of sealed vacuum closure of the at least one receptacle comprising:
positioning on the at least one receptacle a closure material which is capable of being deformed;
placing the at least one receptacle in a flexible bag and;
reducing pressure inside the bag;
sealing the bag, the state of reduced pressure being maintained,
the closure step being carried out under temperature conditions which are linked to the filling level in order to prevent the culture medium from boiling when the pressure is reduced.

2. Method for packaging living cells according to claim 1, **characterised in that** the filling phase is preceded by a step of bringing the culture medium to temperature so that the culture medium has a temperature corresponding to the temperature conditions.

3. Method for packaging cells in a culture according to either claim 1 or claim 2, **characterised in that** the step for reducing the pressure is carried out in order to obtain a pressure in the receptacle which is between approximately 20 millibars and approximately 120 millibars after the closure step.

4. Method for packaging cells in culture according to claim 3, **characterised in that** the step for reducing the pressure is carried out in order to obtain a pressure in the receptacle of approximately 60 millibars after the closure step.

5. Method for packaging cells in culture according to any one of claims 1 to 4, **characterised in that** the step for reducing the pressure is carried out whilst the culture medium has a temperature greater than 0°C and less than approximately 20°C, the at least one receptacle being filled substantially completely with the culture medium.

6. Method for packaging cells in culture according to claim 5, **characterised in that** the step for reducing the pressure is carried out whilst the culture medium has a temperature of approximately 4°C.

7. Method for packaging cells in culture according to any one of claims 1 to 6, **characterised in that** the closure material has a mean hardness of approximately 60 shores.

8. Method for packaging cells in culture according to claim 7, **characterised in that** the closure material is an elastomer material.

9. Method for packaging cells in culture according to any one of claims 1 to 8, **characterised in that** the method comprises a step of covering the closure material with a rigid retention material.

10. Packaging for living cells in culture which are intended to be transported then returned to the culture, the packaging comprising:
at least one receptacle (21) comprising at least one well carried by a culture plate which is closed in a sealed manner by a closure material (22) that is capable of being deformed, the receptacle being at least partially filled with a culture medium that contains the living cells and,
a sealed bag (24) which contains the at least one receptacle or the receptacles (21) being under vacuum in the bag (24).

## Patentansprüche

1. Verfahren zum Verpacken von in Kultur befindlichen lebenden Zellen im Hinblick auf ihren Transport, anschließend ihre Rekultivierung, wobei die Zellen sich in einem Kulturmedium befinden, das in wenigstens einem Behälter, der von wenigstens einem von einer Kulturplatte getragenen Well gebildet ist, enthalten ist, **dadurch gekennzeichnet, dass** es umfasst:
eine Phase zum Befüllen des wenigstens einen Behälters mit dem die lebenden Zellen enthaltenden Kulturmedium entsprechend einem bestimmten Füllungsgrad;
einen Schritt zum dichten Verschließen unter Vakuum des wenigstens einen Behälters, umfassend:
ein Aufbringen eines verformungsfähigen Verschlussmaterials auf dem wenigstens einen Behälter;
ein Einbringen des wenigstens einen Behälters in einen flexiblen Beutel
und
ein Beaufschlagen des Innenraums des Beutels mit Unterdruck,
ein Versiegeln des Beutels, wobei die Unterdruckbeaufschlagung aufrechterhalten wird,
wobei der Schritt des Verschließens unter Temperaturbedingungen, die in Korrelation zu dem Füllungsgrad stehen, durchgeführt wird, um zu vermeiden, dass das Kulturmedium während der Unterdruckbeaufschlagung zum Sieden gebracht wird.

2. Verfahren zum Verpacken von lebenden Zellen nach Anspruch 1, **dadurch gekennzeichnet, dass** der Phase des Befüllens ein Schritt zum Erwärmen des Kulturmediums vorausgeht, so dass das Kulturmedium eine den Temperaturbedingungen entsprechende Temperatur aufweist.

3. Verfahren zum Verpacken von in Kultur befindlichen Zellen nach einem der Ansprüche 1 und 2, **dadurch gekennzeichnet, dass** der Schritt des Unterdruckbeaufschlagens derart vollzogen wird, dass in dem Behälter ein Druck zwischen etwa 20 Millibar und etwa 120 Millibar nach dem Schritt des Verschließens erreicht wird.

4. Verfahren zum Verpacken von in Kultur befindlichen Zellen nach Anspruch 3, **dadurch gekennzeichnet, dass** der Schritt des Unterdruckbeaufschlagens derart vollzogen wird, dass in dem Behälter ein Druck von etwa 60 Millibar nach dem Schritt des Verschließens erreicht wird.

5. Verfahren zum Verpacken von in Kultur befindlichen Zellen nach einem der Ansprüche 1 bis 4, **dadurch gekennzeichnet, dass** der Schritt des Unterdruckbeaufschlagens durchgeführt wird, während das Kulturmedium eine Temperatur von über 0 °C und von unter etwa 20 °C aufweist, wobei der wenigstens eine Behälter im Wesentlichen vollständig mit dem Kulturmedium gefüllt ist.

6. Verfahren zum Verpacken von in Kultur befindlichen Zellen nach Anspruch 5, **dadurch gekennzeichnet, dass** der Schritt des Unterdruckbeaufschlagens durchgeführt wird, während das Kulturmedium eine Temperatur von etwa 4 °C aufweist.

7. Verfahren zum Verpacken von in Kultur befindlichen Zellen nach einem der Ansprüche 1 bis 6, **dadurch gekennzeichnet, dass** das Verschlussmaterial eine mittlere Härte von etwa 60 Shore aufweist.

8. Verfahren zum Verpacken von in Kultur befindlichen Zellen nach Anspruch 7, **dadurch gekennzeichnet, dass** das Verschlussmaterial ein Elastomermaterial ist.

9. Verfahren zum Verpacken von in Kultur befindlichen Zellen nach einem der Ansprüche 1 bis 8, **dadurch gekennzeichnet, dass** es einen Schritt zum Bedecken des Verschlussmaterials mit einem starren Haltematerial umfasst.

10. Verpackung von in Kultur befindlichen lebenden Zellen, die dazu bestimmt sind, transportiert, dann rekultiviert zu werden, wobei die Verpackung wenigstens einen von wenigstens einem von einer Kulturplatte getragenen Well gebildeten Behälter (21), der mit einem verformungsfähigen Verschlussmaterial (22) dicht verschlossen ist, umfasst, wobei der Behälter wenigstens teilweise mit einem die lebenden Zellen enthaltenden Kulturmedium gefüllt ist, sowie einen versiegelten Beutel (24), der den wenigstens einen Behälter oder die Behälter (21 ) enthält, der/die in dem Beutel (24) unter Vakuum ist/sind.
